# FASCICULE DE BREVET EUROPEEN

(11) **EP 2 276 530 B1**
(45) Date de publication et mention de la délivrance du brevet: **12.10.2011**
(21) Numéro de dépôt: 09742296.8
(22) Date de dépôt: 10.04.2009
(51) Int. Cl.: A61M 15/00

(54) **Dispositif de distribution de produit fluide**
Spendevorrichtung für ein flüssiges Produkt
Device for dispensing a fluid product

(30) Priorité: 16.04.2008 FR 0852546
(43) Date de publication de la demande: 26.01.2011
(73) Titulaire: Valois SAS, 27110 Le Neubourg (FR)
(72) Inventeur: SALLAK, Zakaria, 76100 Rouen (FR); COLOMB, Arnaud, F-78480 Verneuil Sur Seine (FR)
(74) Mandataire: CAPRI
(86) Numéro de dépôt international: PCT/FR2009/050645
(87) Numéro de publication internationale: WO 2009/136098

(56) Documents cités:
- WO-A-2008/012458
- FR-A- 2 881 120
- FR-A- 2 896 419
- US-A- 6 012 454

## Description

La présente invention concerne un dispositif de distribution de produit fluide, et plus particulièrement un inhalateur de poudre sèche.

Les inhalateurs de poudre sèche sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blister allongé ou des blisters disposés sur un disque circulaire rotatif. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre évidemment, l'utilisation est plus complexe, puisque l'utilisateur est obligé de charger une capsule dans l'inhalateur avant chaque utilisation. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. En variante, il a été proposé de percer la paroi ou couche de fermeture. Ceci peut présenter l'inconvénient que les parties de paroi découpées risquent de retenir une partie de la dose à l'intérieur du réservoir, la précision et reproductibilité de dosage n'étant donc pas garanties.

Les documents FR-2 881 120, WO 2008/012458, US-6 012 454 et FR-2 896 419 décrivent des dispositifs de l'état de la technique.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un dispositif qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un dispositif qui évite tout risque de sous-dosage, l'ouverture du réservoir, l'expulsion de la dose et le comptage de la dose émise ne se produisant qu'en cas d'inhalation de l'utilisateur. De plus, la présente invention a pour but d'éviter tout risque de perte de dose en l'absence d'inhalation même si l'utilisateur manipule le dispositif.

La présente invention a donc pour objet un dispositif de distribution de produit fluide comportant un corps pourvu d'un orifice de distribution, au moins un réservoir contenant une dose de produit fluide, telle que de la poudre pharmaceutique, des moyens de support mobiles recevant au moins un réservoir et déplaçables entre une position de non-distribution et une position de distribution, des moyens d'ouverture de réservoir pour ouvrir un réservoir lors de l'actionnement desdits moyens d'ouverture, des moyens de chargement pour solliciter lesdits moyens de support mobiles vers ladite position de distribution, lesdits moyens de chargement comprenant un ressort élastiquement déformable, des moyens de blocage pour maintenir lesdits moyens de support mobiles en position de non-distribution, correspondant à une position de blocage chargée desdits moyens de blocage, des moyens de déclenchement par l'inhalation, pour libérer lesdits moyens de blocage et permettre le déplacement desdits moyens de support mobiles, ensemble avec un réservoir, vers ladite position de distribution, au moment de l'inhalation par l'utilisateur, le dispositif comportant des moyens de modification de l'écoulement d'inhalation de l'utilisateur, adaptés en début d'inhalation à utiliser l'écoulement d'inhalation principalement pour actionner lesdits moyens de déclenchement par l'inhalation, et adaptés en fin d'inhalation à utiliser l'écoulement d'inhalation principalement pour expulser la dose de produit fluide contenue dans le réservoir ouvert.

Avantageusement, lesdits moyens de modification de l'écoulement d'inhalation sont adaptés à augmenter la résistance à l'inhalation en début d'inhalation et à diminuer ladite résistance en fin d'inhalation.

Avantageusement, lesdits moyens de modification de l'écoulement d'inhalation sont adaptés à limiter le débit d'écoulement d'inhalation en début d'inhalation et à augmenter ledit débit en fin d'inhalation.

Avantageusement, lesdits moyens de modification de l'écoulement d'inhalation comportent un élément mobile déformable et/ou déplaçable entre une position de bouchage, dans laquelle il bouche un passage d'air d'inhalation, et une position de débouchage, dans laquelle il ne bouche pas ledit passage, ledit élément mobile étant en position de bouchage lorsque les moyens de support mobiles sont en position de non-distribution, et en position de débouchage lorsque les moyens de support mobiles sont en position de distribution.

Avantageusement, ledit passage d'air est formé dans ledit corps et est relié à un embout d'inhalation, ledit élément mobile coopérant d'une part avec ledit corps et d'autre part avec lesdits moyens de support mobiles.

Avantageusement, ledit élément mobile est monté pivotant par rapport audit corps.

Avantageusement, ledit élément mobile est élastiquement déformable par rapport audit corps.

Avantageusement, ledit élément mobile comporte au moins deux branches reliées à une zone centrale formant pivot, une première branche coopérant avec les moyens de support mobiles et une seconde branche coopérant avec ledit passage d'air.

Avantageusement, ledit élément mobile comporte une troisième branche adaptée à s'appuyer et/ou à coulisser contre une partie solidaire dudit corps.

Avantageusement, lesdits moyens de blocage comportent un élément de blocage mobile entre une position de blocage et une position de déblocage, ledit élément de blocage coopérant avec une projection solidaire des moyens de support mobiles, ledit élément de blocage comportant une surface d'appui sensiblement plane sur laquelle vient s'appuyer ladite projection de forme arrondie, un déplacement dudit élément de blocage vers sa position de déblocage provoquant un glissement de ladite projection en direction d'un bord d'extrémité de ladite surface d'appui, à partir duquel les moyens de support mobiles sont déplacés vers leur position de distribution par lesdits moyens de chargement, le point de contact en position de blocage chargée entre la projection et la surface d'appui étant située à une distance non nulle dudit bord d'extrémité.

Avantageusement, ledit élément de blocage est monté pivotant sur le corps.

Avantageusement, la force exercée par les moyens de chargement sur lesdits moyens de support mobiles en position de blocage chargée est comprise entre 5 N et 10 N.

Avantageusement, la force générée par l'inhalation du patient suffisante pour libérer lesdits moyens de blocage est comprise entre 0,05 N et 0,2 N.

Avantageusement, le débit d'inhalation du patient suffisant pour libérer lesdits moyens de blocage est compris entre 10 et 20 l/min.

Avantageusement, le différentiel de pression créé lors de l'inhalation suffisant pour libérer lesdits moyens de blocage est compris entre 4 et 15 mbar.

Avantageusement, le rapport entre la force exercée par les moyens de chargement sur lesdits moyens de support mobiles en position de blocage chargée et la force générée par l'inhalation de l'utilisateur suffisante pour libérer lesdits moyens de blocage est compris entre 80 et 120, notamment environ 100.

Avantageusement, lesdits moyens de blocage comportent une tige reliée d'un côté à des moyens déformables sous l'effet de l'inhalation, et de l'autre côté à l'élément de blocage.

Avantageusement, le dispositif comporte au moins un élément de capot mobile entre une position de fermeture et une position d'ouverture, le déplacement dudit au moins un élément de capot mobile vers sa position d'ouverture amenant ledit dispositif dans sa position de blocage chargée.

Avantageusement, lesdits moyens de déclenchement par l'inhalation comprennent une poche souple.

Avantageusement, le dispositif comprend une pluralité de réservoirs individuels disposés les uns derrière les autres sur une bande souple allongée.

Avantageusement, lesdits moyens d'ouverture de réservoir comportent une aiguille de perçage fixe par rapport au corps.

Ces caractéristiques et avantages et d'autres de la présente invention apparaîtront plus clairement au cours de la description détaillée suivante d'un mode de réalisation de celle-ci, faite en référence aux dessins joints, donnés à titres d'exemples non limitatifs, sur lesquels,
- la figure 1 est une vue schématique partielle en section d'une variante d'un dispositif de distribution de produit fluide, en position armée, avant inhalation,
- la figure 2 est une vue de détail de la figure 1, illustrant un mode de réalisation avantageux de l'invention, avant inhalation, et
- la figure 3 est une vue similaire à la figure 2, après inhalation.

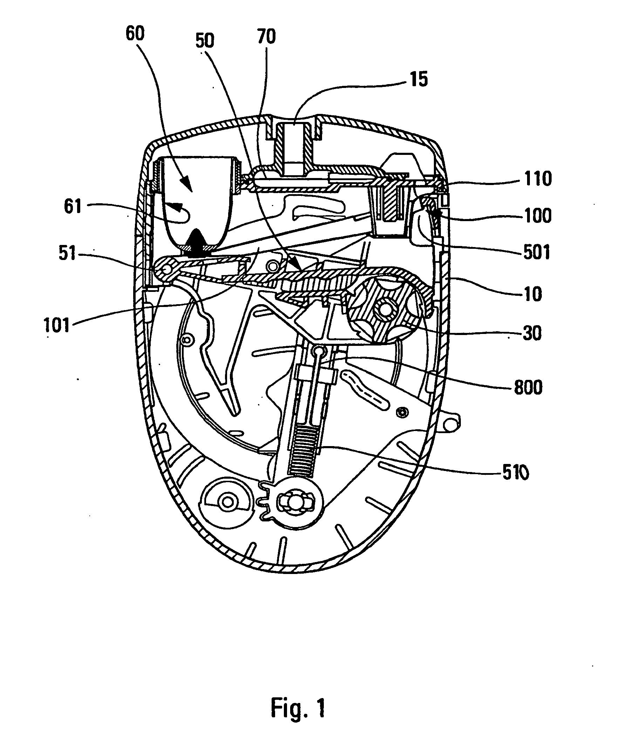

La présente invention concerne de préférence un inhalateur de poudre sèche. Seule une variante de réalisation est représentée sur les figures, étant entendu que de nombreuses autres variantes pourraient être utilisées. Les dessins ne sont donc pas limitatifs. Par ailleurs, de nombreuses caractéristiques de cet inhalateur pourraient aussi être mise en oeuvre pour distribuer du liquide à la place de la poudre. L'inhalateur comporte un corps central 10 sur lequel sont montés coulissants ou pivotants deux éléments ou ailes latérales (non représentés) formant capot lorsque le dispositif est fermé et adaptés à être séparés l'un de l'autre pour ouvrir le dispositif et ainsi charger le dispositif. Le corps 10 peut être de forme environ arrondie en partie basse, et relativement plat en partie haute, comme représenté sur la figure 1, mais il pourrait avoir tout autre forme appropriée. Le corps 10 comporte un orifice de distribution et d'inhalation 15 à travers lequel l'utilisateur va inhaler lors de l'actionnement du dispositif pour créer l'écoulement d'inhalation.

A l'intérieur du corps, il peut être prévu un support de réservoirs individuels (non représentés). Les réservoirs sont avantageusement du type blisters, et le support de réservoir est de préférence une bande souple allongée, sur laquelle les blisters sont disposés les uns derrière les autres, en nombres quelconques appropriés, de manière connue. La bande de blister peut avantageusement être constituée d'une couche ou paroi de base formant des cavités recevant les doses de poudre, et d'une couche ou paroi de fermeture qui obture de manière étanche chacun desdits blisters. La bande de blister peut être enroulée à l'intérieur du corps et des premiers moyens de déplacement de bande 30 sont prévus pour progressivement dérouler cette bande de blister et amener un réservoir individuel ou blister respectif dans une position de distribution à chaque actionnement du dispositif. Lorsqu'un réservoir individuel a été vidé par une inhalation, la partie de bande comportant lesdits réservoirs vides est avantageusement adaptée à s'enrouler dans un autre endroit dudit corps 10.

Des moyens d'ouverture de réservoir 80 sont prévus dans ou solidaires du corps 10, ces moyens d'ouverture comportant des moyens de perçage et/ou de coupage de la couche de fermeture des blisters. Ces moyens d'ouverture ne sont représentés que schématiquement sur les figures.

Des moyens de support mobiles 50 sont adaptés à supporter au moins le réservoir qui est destiné à être ouvert lors de la prochaine inhalation. Ces moyens de supports mobiles 50 formant des seconds moyens de déplacement qui sont adaptés à déplacer le réservoir à vider contre lesdits moyens d'ouverture du dispositif lors de l'actionnement. Avantageusement, ces moyens de supports mobiles 50 sont sollicités directement ou indirectement par des moyens de chargement 800 comprenant un ressort élastiquement déformable 510, une tige ou tout autre élément élastique équivalent, ledit ressort étant préchargé, notamment lors de l'ouverture du dispositif. Avantageusement, les moyens de support mobiles 50 sont déplaçables entre une première position (position de non-distribution) et une seconde position (position de distribution) qui est la position d'ouverture de réservoir et donc d'inhalation. Les moyens de support mobiles 50 sont retenus en position chargée par des moyens de blocage appropriés 100, adaptés à être déclenchés par l'inhalation, comme expliqué ci-après.

Avantageusement, les moyens de support mobiles 50 comportent une pièce sensiblement rigide, telle qu'une tige, articulée ou pivotée autour d'un axe 51 par rapport audit corps 10. Une roue de guidage ou d'indexage 30 fixée de manière rotative sur lesdits moyens de support mobiles 50, reçoit et guide les blisters. Une rotation de cette roue de guidage 30 fait ainsi avancer la bande de blister dans une première direction. Dans une position angulaire particulière, un réservoir ou blister donné est toujours en position pour être ouvert par les moyens d'ouverture.

Cette roue d'indexage 30 forme donc les premiers moyens de déplacement des réservoirs, permettant de faire avancer la bande souple lors de chaque cycle d'actionnement, alors que lesdits moyens de support mobiles 50 forment des seconds moyens de déplacement amenant un réservoir respectif contre lesdits moyens d'ouverture 80 à chaque actionnement.

Lorsque le réservoir se déplace vers sa position d'ouverture pour être ouvert par les moyens d'ouverture 80, ces moyens d'ouverture sont de préférence fixes par rapport au corps 10. Toutefois, il est envisageable que ces moyens d'ouverture puissent également être mobiles pendant la phase d'ouverture du réservoir. Par exemple, les moyens d'ouverture pourraient se déplacer en direction du réservoir pendant que le réservoir se déplace en direction des moyens d'ouverture. Dans une autre variante, on pourrait également envisager que le réservoir et les moyens d'ouverture se déplacent dans la même direction lors de l'actionnement, le réservoir se déplaçant plus rapidement dans cette direction de sorte qu'il vient en contact avec lesdits moyens d'ouverture pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement des moyens 60 déplaçables et/ou déformables sous l'effet de l'inhalation, ces moyens 60 étant adaptés à libérer les moyens de blocage 100. Ces moyens 60 comprennent avantageusement une chambre ou poche d'air déformable 61 coopérant avec les moyens de blocage 100 desdits moyens de support mobiles 50. L'inhalation de l'utilisateur provoque la déformation de ladite chambre d'air déformable 61, permettant ainsi de libérer lesdits moyens de blocage 100 et donc de débloquer les moyens de support mobiles 50 pour permettre le déplacement de la roue de guidage 30, et donc du réservoir à vider, vers sa position d'ouverture. Avantageusement, cette chambre d'air 61 peut être reliée d'une part à l'orifice d'inhalation 15 et d'autre part auxdits moyens de blocage 100 de manière directe ou indirecte. Ainsi, lors de l'inhalation, la chambre 61 se déforme et/ou se contracte, provoquant ainsi le déplacement desdits moyens de blocage 100 dans une position de déblocage. La chambre d'air 61 est reliée à l'orifice d'inhalation 15 via un canal avantageusement disposé autour du canal d'expulsion du produit lui-même relié à une chambre de distribution 70. La chambre 61 peut être fixée à une tige 101 reliée aux moyens de blocage 100, l'inhalation provoquant la déformation de la chambre 61 et donc le pivotement de la tige 101 pour déplacer lesdits moyens de blocage 100.

L'inhalateur comporte en outre une chambre de distribution 70 qui est destinée à recevoir la dose de poudre après ouverture d'un réservoir respectif. Avantageusement, cette chambre de distribution 70 est pourvue d'au moins un élément sensiblement sphérique, tel qu'une bille (non représentée) qui se déplace à l'intérieur de ladite chambre 70 pendant l'inhalation, pour améliorer la distribution du mélange air et poudre après ouverture d'un réservoir, afin d'augmenter l'efficacité du dispositif.

Après l'inhalation, lorsque l'utilisateur referme le dispositif, tous les composants reviennent vers leur position de repos initiale, à savoir que les moyens de support mobiles 50 pivotent par rapport à leur axe de pivotement en retour vers leur position de non distribution en éloignement des moyens d'ouverture de réservoir, et l'élément de chargement est également ramené dans sa position de repos initiale dans laquelle il n'est pas comprimé ou déformé. Le dispositif est alors prêt pour un nouveau cycle d'utilisation.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

Comme expliqué ci-dessus, des moyens de blocage 100 sont prévus pour retenir lesdits moyens de support mobiles 50 dans ladite position de non distribution, correspondant à une position de blocage chargée des moyens de blocage. Lesdits moyens de blocage 100 comportent avantageusement un élément de blocage 110 adapté à coopérer avec une extension ou projection 501 desdits moyens de support mobiles 50. Lorsque la projection n'est plus retenue, les moyens de support mobiles 50 peuvent se déplacer vers leur position de distribution sous l'effet de la force exercée sur eux par les moyens de chargement 800. Lesdits moyens de blocage 100 sont avantageusement connectés à la chambre déformable 61 sensible à l'inhalation de l'utilisateur par l'intermédiaire d'une tige 101, de sorte que lors de l'inhalation de l'utilisateur, ladite chambre se déforme, faisant ainsi pivoter la tige 101 et par la même ledit élément de blocage 110, libérant ainsi l'extension 501. Ceci permet le déplacement desdits moyens de support mobiles 50 vers leur position de distribution sous l'effet de la force exercée par le ressort comprimé 510. Ce déplacement des moyens de support mobiles 50 provoque l'ouverture d'un réservoir individuel comme décrit précédemment.

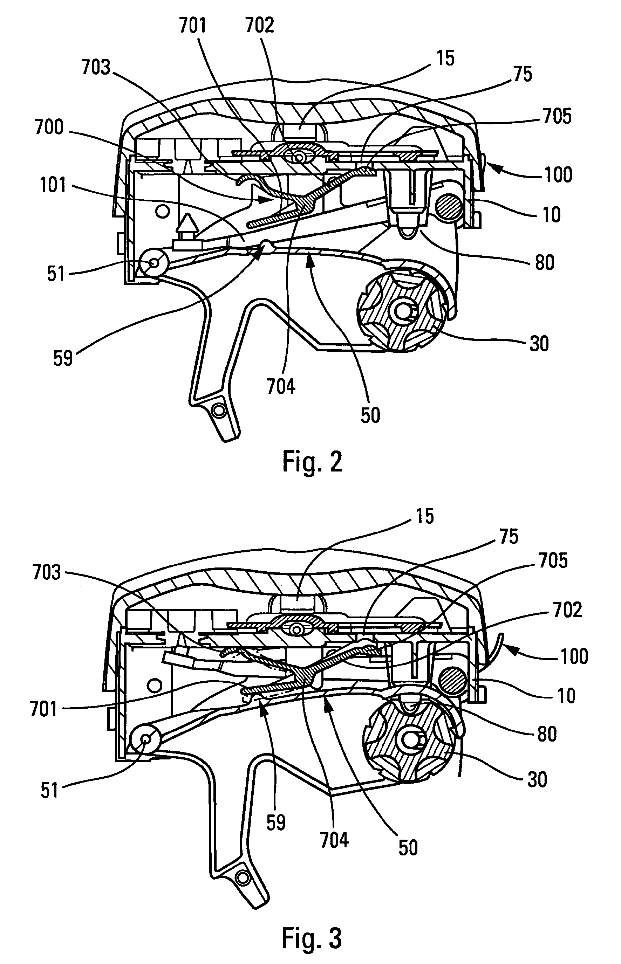

Après l'inhalation, c'est-à-dire en position de distribution représentée sur la figure 3, les moyens de blocage 100 ont pivoté et les moyens de support mobiles 50 ont été déplacés vers le haut par le ressort comprimé 510. Le pivotement des moyens de blocage, en particulier de l'élément de blocage 110, peut provoquer la sortie hors du corps 10 d'une partie d'extrémité de cet élément de blocage 110, comme visible sur la figure 3. Lorsque l'utilisateur referme ensuite les éléments de capot mobile, l'un des éléments de capot vient en fin de fermeture pousser sur ladite partie d'extrémité ce qui ramène les moyens de blocage 100 vers leur position initiale avec la chambre 61 qui est également ramené dans sa position initiale, comme visible notamment sur la figure 5.

Ainsi, lorsque l'utilisateur ouvre l'inhalateur il charge le système. S'il n'inhale pas et qu'il referme l'inhalateur, celui-ci reprend simplement sa position de départ sans déplacer les réservoirs ni les moyens de blocage 100. Il n'y a donc aucun risque qu'un réservoir (et donc une dose de produit actif) soit perdu par un actionnement malencontreux ou incomplet dans lequel l'utilisateur n'exercerait pas d'inhalation entre l'ouverture et la fermeture. L'ouverture du réservoir, son vidage, la distribution de la poudre dans les poumons de l'utilisateur ainsi que le déplacement de la bande de blisters pour amener un nouveau réservoir plein en face des moyens d'ouverture n'est donc possible que si l'utilisateur inhale.

Ainsi, les moyens de blocage doivent être mis sous contrainte pour se libérer. Une précharge est donc appliquée sur le mécanisme. L'inhalation du patient provoque la déformation de la chambre déformable 61 qui entraîne en rotation l'élément de blocage 110 et libère le mécanisme. Un avantage de ces moyens de blocage est de pouvoir fonctionner avec un rapport de 100 entre la précharge appliquée et l'effort nécessaire pour déformer le diaphragme. En effet, 0,05N à 0,2N sont généralement suffisants pour la déformation du diaphragme (effort généré par l'inhalation patient) malgré une précharge de 5 à 10N appliquée sur le mécanisme (cette précharge permet de garantir un effort de perçage suffisant pour permettre à l'aiguille de pénétrer dans le blister). Il suffit au patient de générer 4mbar à 15mbar de différentiel de pression (pression correspondant à un débit compris entre 10 litres par minute et 20 litres par minute) pour déclencher le mécanisme: ce débit d'inhalation est un débit jugé confortable pour un patient asthmatique ou atteint d'une maladie chronique de voies pulmonaires. Le débit généré permettra à l'issu du perçage d'entraîner la poudre vers les bronches du patient si le débit minimum d'inhalation n'est pas atteint, aucune dose ne sera libéré: ce dispositif de prévention empêche ainsi le déclenchement intempestif du device.

Un partage judicieux des flux d'air permet de garantir le fonctionnement des moyens de blocage (1/2 à 2/3 du flux global), tout en atteignant les performances de distribution de poudre voulues (1/3 à 1/2 du flux global).

Le réarmement des moyens de blocage peut être assuré par l'élément de blocage 110, ou en variante par un ressort de torsion, ou même par l'élasticité de la chambre 61. Ce réarmement permet au mécanisme de revenir à son état initial après chaque cycle d'inhalation dès fermeture de l'inhalateur, de manière répétable.

De manière avantageuse, en cas d'ouverture et fermeture du dispositif sans inhalation, le système reste au repos. Il n'y a donc pas de risque de surdosage. De plus, aucune précharge n'est appliquée lorsque l'inhalateur n'est pas ouvert, ce qui favorise la stabilité des composants au cours du temps. Avantageusement, en position fermée de l'inhalateur, la projection 501 est disposée à une distance non nulle de l'élément de blocage pour assurer le réarmement du système.

Selon l'invention, le dispositif comporte des moyens de modification de l'écoulement d'inhalation, adaptés à limiter l'écoulement d'inhalation en début d'inhalation, puis à augmenter ledit écoulement d'inhalation en fin d'inhalation. Dans l'exemple représenté sur les figures 2 et 3, ces moyens comportent un élément mobile 700 déformable et/ou déplaçable entre une position de bouchage (figure 2) et une position de débouchage (figure 3). En position de bouchage, l'élément mobile 700 bouche ou obture, via une extension 705, un passage d'air 75 avantageusement formé dans une partie du corps 10 reliée à l'embout d'inhalation. La totalité ou presque de l'écoulement d'inhalation est alors disponible pour déformer la chambre déformable 61. Par contre, en position de débouchage, le passage d'air 75 n'est plus obturé, permettant ainsi de réduire la résistance à l'inhalation du patient, ce qui améliore le confort de l'utilisateur. Ceci permet aussi d'optimiser le flux d'inhalation pour expulser la dose de poudre hors du réservoir ouvert. Avant l'ouverture du réservoir, la majorité du flux sert à déclencher le système, et après ouverture, la majorité du flux sert à expulser la dose. Ceci permet aussi d'octroyer une résistance variable au système de déclenchement de l'inhalation. Ainsi, le fait de boucher le passage d'air 75 permet d'anticiper le moment du déclenchement et de diminuer la plage de fonctionnement des moyens de blocage. En d'autres termes, pour une même variation de pression de déclenchement, ou une même force de déclenchement mécanique, la plage de débit d'inhalation est réduite, ce qui provoque une augmentation temporaire de la résistance, tout en augmentant la substance industrielle. Après ouverture du passage d'air, le débit peut être augmenté pour favoriser un bon vidage du réservoir ouvert.

L'élément mobile 700 est en position de bouchage lorsque les moyens de support mobiles 50 sont en position de non-distribution, et en position de débouchage lorsque les moyens de support mobiles 50 se déplacent en position de distribution. Avantageusement, l'élément mobile 700 comporte une partie, de préférence une première branche 701, qui coopère avec lesdits moyens de support mobiles 50, de préférence avec une projection 59 de ceux-ci, adaptée à déformer et/ou déplacer une autre partie, de préférence une seconde branche 702, de l'élément mobile 700 de sa position de bouchage vers sa position de débouchage, comme visible sur la figure 3. Les première et seconde branches 701 et 702 sont de préférence reliées à une zone centrale formant pivot 704. Ces branches peuvent être pivotantes et/ou élastiquement déformables par rapport à ladite zone 704, de sorte que l'élément mobile dans son ensemble peut être pivotant et/ou élastiquement déformable par rapport au corps 10. Avantageusement, une troisième branche 703 est reliée à ladite zone centrale 704, adaptée à coopérer, notamment glisser, sur une partie solidaire du corps 10, pour assurer un guidage de l'élément mobile et une meilleure stabilité du système.

Bien entendu, l'invention n'est pas limitée à l'élément mobile représenté sur les figures 2 et 3, particulièrement adapté au système de déclenchement représenté, et les moyens de modification de l'écoulement d'inhalation pourraient être différents de ceux représentés.

En général, la bande de blister est formée par une bande présentant deux extrémités. En variante, on pourrait utiliser une bande continue. D'autres modifications sont également possibles sans sortir du cadre de la présente invention.

La présente invention permet donc de fournir un inhalateur de poudre sèche qui procure notamment les fonctions suivantes :
■ une pluralité de doses individuelles de poudre stockées dans des réservoirs individuels étanches, par exemple 30 ou 60 doses stockées sur une bande enroulée en bobine ;
■ la poudre libérée au moyen d'un perçage actionné par l'inhalation de l'utilisateur, ce perçage du blister étant réalisé au moyen d'un système de détection d'inhalation couplé à un système de libération préchargé ;
■ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
■ des moyens pour éviter une perte de dose en cas d'ouverture de l'inhalateur mais en l'absence d'inhalation. Dans ce cas, lors de la fermeture de l'inhalateur, le dispositif revient exactement à sa position de départ.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

L'inhalateur de l'invention, incorporant toutes ou parties des fonctions décrites, s'avère fournir des performances supérieures à celles des dispositifs existants. En particulier, l'inhalateur de l'invention fournit de préférence un taux de vidage des réservoirs d'au moins 90% à chaque actionnement. Avantageusement, ce taux de vidage, correspondant au pourcentage du produit fluide expulsé hors d'un réservoir ouvert lors d'un actionnement du dispositif, est supérieur à 95%, de préférence même supérieur à 97%. Ce taux de vidage élevé est notamment même supérieur aux performances obtenues avec des inhalateurs actifs qui sont généralement plus efficaces que les inhalateurs passifs, et dans lesquels ce n'est pas l'écoulement d'inhalation qui vide le blister et expulse la dose mais un écoulement d'air comprimé libéré au moment de l'inhalation. Il garantit une efficacité maximale du dispositif de l'invention. Couplé à l'ouverture déclenchée par l'inhalation, ce taux de vidage élevé garantit une distribution optimale du produit fluide, en l'occurrence la poudre, dans les poumons de l'utilisateur. Par ailleurs, l'invention fournit également une meilleure régularité de vidage des réservoirs lors d'actionnements successifs. Ainsi, en se référant par exemple à 10 réservoirs d'une bande de blisters, il s'avère que le taux de vidage varie de moins de 15%, avantageusement de moins de 10%, de préférence de moins de 5% d'un réservoir à l'autre. Cette meilleure régularité garantit une meilleure reproductibilité de la dose et donc aussi une meilleure efficacité du dispositif de l'invention.

Diverses modifications sont également possibles pour un homme du métier sans sortir du cadre de la présente invention tel que défini par les revendications annexées.

## Revendications

1. Dispositif de distribution de produit fluide comportant :
- un corps (10) pourvu d'un orifice de distribution (15),
- au moins un réservoir contenant une dose de produit fluide, telle que de la poudre pharmaceutique,
- des moyens de support mobiles (50) recevant au moins un réservoir et déplaçables entre une position de non-distribution et une position de distribution,
- des moyens d'ouverture de réservoir (80) pour ouvrir un réservoir lors de l'actionnement desdits moyens d'ouverture,
- des moyens de chargement (800) pour solliciter lesdits moyens de support mobiles vers ladite position de distribution, lesdits moyens de chargement comprenant un ressort élastiquement déformable (510),
- des moyens de blocage (100) pour maintenir lesdits moyens de support mobiles en position de non-distribution, correspondant à une position de blocage chargée desdits moyens de blocage,
- des moyens de déclenchement par l'inhalation (60), pour libérer lesdits moyens de blocage et permettre le déplacement desdits moyens de support mobiles, ensemble avec un réservoir, vers ladite position de distribution, au moment de l'inhalation par l'utilisateur,
**caractérisé en ce que** le dispositif comporte des moyens de modification de l'écoulement d'inhalation de l'utilisateur (700), adaptés en début d'inhalation à utiliser l'écoulement d'inhalation principalement pour actionner lesdits moyens de déclenchement par l'inhalation (60), et adaptés en fin d'inhalation à utiliser l'écoulement d'inhalation principalement pour expulser la dose de produit fluide contenue dans le réservoir ouvert.

2. Dispositif selon la revendication 1, dans lequel lesdits moyens de modification de l'écoulement d'inhalation (700) sont adaptés à augmenter la résistance à l'inhalation en début d'inhalation et à diminuer ladite résistance en fin d'inhalation.

3. Dispositif selon la revendication 1 ou 2, dans lequel lesdits moyens de modification de l'écoulement d'inhalation (700) sont adaptés à limiter le débit d'écoulement d'inhalation en début d'inhalation et à augmenter ledit débit en fin d'inhalation.

4. Dispositif selon l'une quelconque des revendications précédentes, dans lequel lesdits moyens de modification de l'écoulement d'inhalation comportent un élément mobile (700) déformable et/ou déplaçable entre une position de bouchage, dans laquelle il bouche un passage d'air d'inhalation (75), et une position de débouchage, dans laquelle il ne bouche pas ledit passage (75), ledit élément mobile (700) étant en position de bouchage lorsque les moyens de support mobiles (50) sont en position de non-distribution, et en position de débouchage lorsque les moyens de support mobiles (50) sont en position de distribution.

5. Dispositif selon la revendication 4, dans lequel ledit passage d'air (75) est formé dans ledit corps (10) et est relié à un embout d'inhalation, ledit élément mobile (700) coopérant d'une part avec ledit corps (10) et d'autre part avec lesdits moyens de support mobiles (50).

6. Dispositif selon la revendication 4 ou 5, dans lequel ledit élément mobile (700) est monté pivotant par rapport audit corps (10).

7. Dispositif selon l'une quelconque des revendications 4 à 6, dans lequel ledit élément mobile (700) est élastiquement déformable par rapport audit corps (10).

8. Dispositif selon l'une quelconque des revendications 4 à 7, dans lequel ledit élément mobile (700) comporte au moins deux branches (701, 702, 703) reliées à une zone centrale formant pivot (704), une première branche (701) coopérant avec les moyens de support mobiles (50) et une seconde branche (702) coopérant avec ledit passage d'air (75).

9. Dispositif selon la revendication 8, dans lequel ledit élément mobile (700) comporte une troisième branche (703) adaptée à s'appuyer et/ou à coulisser contre une partie solidaire dudit corps (10).

10. Dispositif selon l'une quelconque des revendications précédentes, dans lequel lesdits moyens de blocage (100) comportent un élément de blocage (110) mobile entre une position de blocage et une position de déblocage, ledit élément de blocage (110) coopérant avec une projection (501) solidaire des moyens de support mobiles (50), ledit élément de blocage (110) comportant une surface d'appui (111) sensiblement plane sur laquelle vient s'appuyer ladite projection (501) de forme arrondie, un déplacement dudit élément de blocage (110) vers sa position de déblocage provoquant un glissement de ladite projection (501) en direction d'un bord d'extrémité (112) de ladite surface d'appui (111), à partir duquel les moyens de support mobiles (50) sont déplacés vers leur position de distribution par lesdits moyens de chargement, le point de contact en position de blocage chargée entre la projection (501) et la surface d'appui (111) étant située à une distance non nulle (B) dudit bord d'extrémité (112).

11. Dispositif selon la revendication 10, dans lequel ledit élément de blocage (110) est monté pivotant sur le corps (10).

12. Dispositif selon l'une quelconque des revendications précédentes, dans lequel les moyens de chargement exerçant sur lesdits moyens de support mobiles (50), en position de blocage chargée, une force comprise entre 5 N et 10 N.

13. Dispositif selon l'une quelconque des revendications précédentes, dans lequel l'inhalation du patient génère une force comprise entre 0,05 N et 0,2 N pour libérer lesdits moyens de blocage (100).

14. Dispositif selon l'une quelconque des revendications précédentes, dans lequel l'inhalation du patient génère un débit d'inhalation du patient compris entre 10 et 20 l/min pour libérer lesdits moyens de blocage (100).

15. Dispositif selon l'une quelconque des revendications précédentes, dans lequel l'inhalation du patient génère un différentiel de pression compris entre 4 et 15 mbar pour libérer lesdits moyens de blocage (100).

16. Dispositif selon l'une quelconque des revendications précédentes, dans lequel le rapport entre la force exercée par les moyens de chargement sur lesdits moyens de support mobiles (50) en position de blocage chargée et la force générée par l'inhalation de l'utilisateur suffisante pour libérer lesdits moyens de blocage (100) est compris entre 80 et 120, notamment environ 100.

17. Dispositif selon l'une quelconque des revendications précédentes, dans lequel lesdits moyens de blocage (100) comportent une tige (101) reliée d'un côté à des moyens déformables sous l'effet de l'inhalation (60), et de l'autre côté à l'élément de blocage (110).

18. Dispositif selon l'une quelconque des revendications précédentes, comportant au moins un élément de capot (11, 12) mobile entre une position de fermeture et une position d'ouverture, le déplacement dudit au moins un élément de capot mobile vers sa position d'ouverture amenant ledit dispositif dans sa position de blocage chargée.

19. Dispositif selon l'une quelconque des revendications précédentes, dans lequel lesdits moyens de déclenchement par l'inhalation (60) comprennent une poche souple (61).

20. Dispositif selon l'une quelconque des revendications précédentes, comprenant une pluralité de réservoirs individuels disposés les uns derrière les autres sur une bande souple allongée.

21. Dispositif selon l'une quelconque des revendications précédentes, dans lequel lesdits moyens d'ouverture de réservoir comportent une aiguille de perçage (80) fixe par rapport au corps (10).

## Claims

1. A fluid dispenser device comprising:
· a body (10) provided with a dispenser orifice (15);
· at least one reservoir containing a dose of fluid, such as pharmaceutical powder;
· movable support means (50) that receive at least one reservoir, and that are displaceable between a non-dispensing position and a dispensing position;
· reservoir-opening means (80) for opening a reservoir when said opening means are actuated;
· stressing means (800) for urging said movable support means towards said dispensing position, said stressing means including an elastically-deformable spring (510);
· blocking means (100) for retaining said movable support means in the non-dispensing position corresponding to a cocked position of said blocking means; and
· inhalation trigger means (60) for releasing said blocking means and for enabling said movable support means, together with a reservoir, to be displaced towards said dispensing position at the time a user inhales;
the device being **characterized in that** it includes means (700) for modifying the inhalation flow of the user, which means, at the start of inhalation, are adapted to use the inhalation flow mainly to actuate said inhalation trigger means (60), and, at the end of inhalation, are adapted to use the inhalation flow mainly to expel the dose of fluid contained in the open reservoir.

2. A device according to claim 1, wherein said means (700) for modifying the inhalation flow are adapted to increase the resistance to inhalation at the start of inhalation, and to decrease said resistance at the end of inhalation.

3. A device according to claim 1 or claim 2, wherein said means (700) for modifying the inhalation flow are adapted to limit the inhalation flow rate at the start of inhalation, and to increase said rate at the end of inhalation.

4. A device according to any preceding claim, wherein said means for modifying the inhalation flow comprise a movable element (700) that is deformable and/or displaceable between a plugging position in which it plugs an inhalation air passage (75), and an unplugging position in which it does not plug said passage (75), said movable element (700) being in its plugging position when the movable support means (50) are in the non-dispensing position, and in its unplugging position when the movable support means (50) are in the dispensing position.

5. A device according to claim 4, wherein said air passage (75) is formed in said body (10) and is connected to an inhalation endpiece, said movable element (700) co-operating firstly with said body (10) and secondly with said movable support means (50).

6. A device according to claim 4 or claim 5, wherein said movable element (700) is pivotally mounted relative to said body (10).

7. A device according to any one of claims 4 to 6, wherein said movable element (700) is elastically deformable relative to said body (10).

8. A device according to any one of claims 4 to 7, wherein said movable element (700) includes at least two branches (701, 702, 703) that are connected to a pivot-forming central zone (704), a first branch (701) co-operating with the movable support means (50), and a second branch (702) co-operating with said air passage (75).

9. A device according to claim 8, wherein said movable element (700) includes a third branch (703) that is adapted to bear and/or to slide against a portion that is fixed relative to said body (10).

10. A device according to any preceding claim, wherein said blocking means (100) comprise a blocking element (110) that is movable between a blocking position and an unblocking position, said blocking element (110) co-operating with a projection (501) that is secured to movable support means (50), said blocking element (110) including a substantially plane bearing surface (111) against which said projection (501) of rounded shape comes to bear, displacement of said blocking element (110) towards its unblocking position causing said projection (501) to slide towards an end edge (112) of said bearing surface (111), from which position the movable support means (50) are displaced towards their dispensing position by said stressing means, the contact point in the cocked position between the projection (501) and the bearing surface (111) being situated at a non-zero distance (B) from said end edge (112).

11. A device according to claim 10, wherein said blocking element (110) is pivotally mounted on the body (10).

12. A device according to any preceding claim, wherein the stressing means exert, on said movable support means (50) in the cocked position, a force lying in the range 5 newtons (N) to 10 N.

13. A device according to any preceding claim, wherein the inhalation of the patient generates a force lying in the range 0.05 N to 0.2 N in order to release said blocking means (100).

14. A device according to any preceding claim, wherein the inhalation of the patient generates an inhalation rate of the patient lying in the range 10 L/min to 20 L/min in order to release releasing said blocking means (100).

15. A device according to any preceding claim, wherein the inhalation of the patient generates a pressure difference lying in the range 4 mbar to 15 mbar in order to release said blocking means (100).

16. A device according to any preceding claim, wherein the ratio between the force exerted by the stressing means on said movable support means (50) in the cocked position and the force generated by inhalation of the user that is sufficient to release said blocking means (100) lies in the range 80 to 120, in particular about 100.

17. A device according to any preceding claim, wherein said blocking means (100) comprise a rod (101) that is connected at one end to means (60) that are deformed under the effect of inhalation, and that is connected at its other end to the blocking element (110).

18. A device according to any preceding claim, including at least one cover element (11, 12) that is movable between a closed position and an open position, the displacement of said at least one movable cover element towards its open position bringing said device into its cocked position.

19. A device according to any preceding claim, wherein said inhalation trigger means (60) comprise a flexible pouch (61).

20. A device according to any preceding claim, including a plurality of individual reservoirs disposed one behind another on an elongate flexible strip.

21. A device according to any preceding claim, wherein said reservoir-opening means comprise a perforator needle (80) that is stationary relative to the body (10).

## Patentansprüche

1. Spendevorrichtung für ein flließfähiges Produkt, aufweisend:
- einen Körper (10), der mit einer Abgabeöffnung (15) versehen ist,
- zumindest einen Vorratsbehälter, der eine Dosis flüssiges Produkt, wie etwa pharmazeutisches Pulver enthält,
- bewegliche Tragmittel (50), die zumindest einen Vorratsbehälter aufnehmen und zwischen einer Nichtabgabestellung und einer Abgabestellung verlagerbar sind,
- Vorratsbehälter-Öffnungsmittel (80) zum Öffnen eines Vorratsbehälters bei der Betätigung der Öffnungsmittel,
- Spannmittel (800) zum Spannen der beweglichen Tragmittel in Richtung auf die Abgabestellung, wobei die Spannmittel eine elastisch verformbare Feder (510) aufweisen,
- Blockiermittel (100) zum Halten der Tragmittel in der Nichtabgabestellung entsprechend einer gespannten Blockierstellung der Blockiermittel,
- Auslösemittel (60) durch Inhalation zum Freigeben der Blockiermittel und zum Ermöglichen einer Verlagerung der beweglichen Tragmittel zusammen mit einem Vorratsbehälter in Richtung auf die Abgabestellung zum Zeitpunkt der Inhalation durch den Nutzer,
- **dadurch gekennzeichnet, dass** die Vorrichtung Mittel (700) zur Modifikation des Inhalationsverlaufs des Nutzers aufweist, die dazu ausgelegt sind, zu Beginn der Inhalation die Inhalationshauptströmung zur Betätigung der Auslösemittel (60) durch Inhalation zu nutzen sowie dazu, zum Ende der Inhalation die Inhalationshauptströmung zum Ausstoßen der in dem offenen Vorratsbehälter enthaltenen Dosis fließfähigen Produkts zu nutzen.

2. Vorrichtung nach Anspruch 1, wobei die Mittel (700) zur Modifikation der Inhalationsströmung dazu ausgelegt sind, den Inhalationswiderstand zu Beginn der Inhalation zu erhöhen und zum Ende der Inhaltion herabzusetzen.

3. Vorrichtung nach Anspruch 1 oder 2, wobei die Mittel (700) zur Modifikation der Inhalationsströmung dazu ausgelegt sind, den Inhalationswiderstand zu Beginn der Inhalation zu begrenzen und zum Ende der Inhaltion den Durchsatz zu erhöhen.

4. Vorrichtung nach einem der vorangehenden Ansprüche, wobei die Mittel (700) zur Modifikation der Inhalationsströmung ein bewegliches Element (700) aufweisen, das zwischen einer Sperrstellung, in der es einen Inhalationsluftdurchlass (75) versperrt, und einer Entsperrungsstellung, in der es den Durchlass (75) nicht versperrt, verformbar und/oder verlagerbar ist, wobei das bewegliche Element (700) sich in der Sperrstellung befindet, wenn die beweglichen Tragmittel (50) sich in der Nichtabgabestellung befinden, und in der Entsperrungsstellung, wenn die beweglichen Tragmittel (50) sich in der Abgabestellung befinden.

5. Vorrichtung nach Anspruch 4, wobei der Luftdurchlass (75) in dem Körper (10) gebildet und mit einem Inhalationsmundstück verbunden ist, wobei das bewegliche Element (700) einerseits mit dem Körper (10) und andererseits mit den beweglichen Tragmitteln (50) zusammenwirkt.

6. Vorrichtung nach Anspruch 4 oder 5, wobei das bewegliche Element (700) in Bezug auf den Körper (10) schwenkbar angebracht ist.

7. Vorrichtung nach einem der Ansprüche 4 bis 6, wobei das bewegliche Element (700)) in Bezug auf den Körper (10) elastisch verformbar ist.

8. Vorrichtung nach einem der Ansprüche 4 bis 7, wobei das bewegliche Element (700) zumindest zwei Arme (701, 702, 703) aufweist, die mit einer zentralen Zone verbunden sind, die ein Drehgelenk (704) bildet, wobei ein Arm (701) mit den beweglichen Tragmitteln (700) und ein zweiter Arm (702) mit dem Luftdurchlass (75) zusammenwirkt.

9. Vorrichtung nach Anspruch 8, wobei das bewegliche Element (700) einen dritten Arm (703) aufweist, der dazu ausgelegt ist, sich an einem mit dem Körper (10) fest verbundenen Teil anzulegen und/oder gegen diesen zu gleiten.

10. Vorrichtung nach einem der vorangehenden Ansprüche, wobei die Blockiermittel (100) ein Blockierelement (110) aufweisen, das zwischen einer Blockierstellung und einer Blockadeaufhebestellung beweglich ist, wobei das Blockierelement (110) mit einem Vorsprung (501) zusammenwirkt, der mit den beweglichen Tragmittleln (50) fest verbunden ist, wobei das Blockierelement (110) eine im wesentlichen ebene Anlagefläche (111) aufweist, auf welcher der verrundete Form aufweisende Vorsprung (501) in Anlage gelangt, wobei eine Verlagerung des Blockierelements (110) in Richtung auf seine Blockadeaufhebestellung eine Gleitbewegung des Vorsprungs (501) in Richtung auf einen Außenrand (112) der Anlagefläche (111) auslöst, ausgehend von dem die beweglichen Tragmittel (50) durch die Spannmittel in Richtung auf ihre Ausgabestellung verlagert werden, wobei der Kontaktpunkt in der gespannten Blockierstellung zwischen dem Vorsprung (501) und der Anlagefläche (111) unter einem nicht null betragenden Abstand (B) vom Außenrand (112) zu liegen kommt.

11. Vorrichtung nach Anspruch 10, wobei das Blockierelement (110) auf dem Körper (10) verschwenkbar angebracht ist.

12. Vorrichtung nach einem der vorangehenden Ansprüche, wobei die Spannmittel auf die beweglichen Tragmittel (50) in der gespannten Blockierstellung eine Kraft zwischen 5N und 10N ausüben.

13. Vorrichtung nach einem der vorangehenden Ansprüche, wobei die Inhalation des Patienten eine Kraft zwischen 0,05N und 0,2N erzeugt, um die Blockiermittel (100) frei zu geben.

14. Vorrichtung nach einem der vorangehenden Ansprüche, wobei die Inhalation des Patienten einen Durchsatz zwischen 10 und 20 l/min erzeugt, um die Blockiermittel (100) frei zu geben.

15. Vorrichtung nach einem der vorangehenden Ansprüche, wobei die Inhalation des Patienten eine Druckdifferenz zwischen 4 und 15mbar erzeugt, um die Blockiermittel (100) frei zu geben.

16. Vorrichtung nach einem der vorangehenden Ansprüche, wobei das Verhältnis zwischen der durch die Spannmitel auf die beweglichen Tragmittel (50) in der Blockierstellung ausgebübten Kraft zu der durch die Inhalation des Nutzers erzeugten, zur Freisetzung der Blockiermittel (100) ausreichenden Kraft zwischen 80 und 120, insbesondere in etwa 100 beträgt.

17. Vorrichtung nach einem der vorangehenden Ansprüche, wobei die Blockiermittel (100) eine Stange (101) umfassen, die einerseits mit den unter der Wirkung der Inhalation verformbaren Mitteln (60) und andereseits mit dem Blockierelement (110) verbunden ist.

18. Vorrichtung nach einem der vorangehenden Ansprüche, aufweisend zumindest ein Deckelelement (11, 12), das zwischen einer Schließ-und einer Öffnungsstellung beweglich ist, wobei die Verlagerung des zumindest einen beweglichen Elements in Richtung auf seine Öffnungsstellung die Vorrichtung in ihre gespannte Blockierstellung mitnimmt.

19. Vorrichtung nach einem der vorangehenden Ansprüche, wobei die Mittel (60) zur Auslösung durch Inhalation eine nachgiebige Tasche (61) aufweisen.

20. Vorrichtung nach einem der vorangehenden Ansprüche, aufweisend mehrere einzelene Vorratsbehälter, die hintereinander auf einem nachgiebigen, langgestreckten Band angeordnet sind.

21. Vorrichtung nach einem der vorangehenden Ansprüche, wobei die Vorratsbehälter-Öffnungsmittel eine Stechnadel (80) aufweisen, die in Bezug auf den Körper (10) feststeht.
